# EUROPEAN PATENT SPECIFICATION

(11) **EP 1 537 827 B1**
(45) Date of publication and mention of the grant of the patent: **02.09.2009**
(21) Application number: 04028371.5
(22) Date of filing: 30.11.2004
(51) Int. Cl.: A61B 17/22

(54) **Wire for removing foreign matter in blood vessel and medical device using the wire**
Draht zum Entfernen von Fremdstoffen in einem Blutgefäss und diesen Draht verwendende medizinische Vorrichtung
Câble permettant d'évacuer un corps étranger dans un vaisseau sanguin et dispositif médical utilisant le câble

(30) Priority: 01.12.2003 JP 2003402117
(43) Date of publication of application: 08.06.2005
(73) Proprietor: TERUMO KABUSHIKI KAISHA, Tokyo 151-0072 (JP)
(72) Inventor: Kanamaru, Takeshi, Ashigarakami-gun Kanagawa 259-0151 (JP)
(74) Representative: TBK-Patent

(56) References cited:
- EP-A- 1 273 268
- WO-A-02/054942
- WO-A2-02/092148
- WO-A2-02/094111
- US-A- 5 011 488
- US-A- 5 968 057

## Description

The present invention relates to a wire for removing foreign matter such as an embolus in a blood vessel and a medical device using the wire.

According to the dynamic statistics of population by the Ministry of Health, Labour and Welfare, the causes of death of the Japanese are cancer as the first rank, heart disease as the second rank, and cerebral apoplexy as the third rank. In particular, the death or aftereffect due to cerebral apoplexy has increased and the establishment of a curing method therefor has become urgent.

In recent years, in the treatment for cerebral apoplexy, thrombolytic therapy using a thrombolytic agent for the treatment for acute-phase cerebral infarction has been developed and a curing effect has been exhibited. However, the limit of this effect has been pointed out. That is, doctor's experiences show that a long period of time is required for the dissolution of a thrombus, that a thrombus reduced in size travels in a blood vessel to form a new blocking portion, and that there exists a thrombus that cannot be dissolved by a thrombolytic agent.

In the case of cerebral infarction, it has been demonstrated in the United States and Europe that if a bloodstream is recovered within three hours after the occurrence of infarction, the probability of lifesaving can be increased and the aftereffect due to infarction can also be reduced. Thus, it is strongly demanded to develop a medical device which can be inserted into a cerebral blood vessel to directly and promptly remove a thrombus.

As an example of such a medical device, there is disclosed a wire for removing foreign matter in a blood vessel, wherein the wire includes a wire body, at least two branched wire portions, and a plurality of filament portions. The branched wire portions and the filament portions constitute a foreign matter catching portion for catching the foreign matter in the blood vessel (see Japanese Patent Laid-open No. 2003-10193 corresponding to US Patent Laid-open No. 2003-18355, for example).

However, the conventional wire has a problem such that the foreign matter cannot be reliably caught in some cases as depending upon the size of the foreign matter. For example, when the size of the foreign matter is larger than that of the foreign matter catching portion, there is a case that the foreign matter may fall from the foreign matter catching portion during the catching operation. In such a case, an operation for replacing the smaller wire by a larger wire according to the size of the foreign matter, for example, may be required to cause a troublesome manipulation.

D1 (WO 02/094111) discloses a vascular device for emboli and thrombi removal having two holding portions. A first holding portion is supposed to catch the foreign matter when being retracted into the lumen. After the holding portion together with the foreign matter is received in the lumen, a second holding portion serves to catch leftover particulate from the foreign matter which was affected by the retraction into the lumen.

D2 (US 5011488) discloses a thrombus extraction system having two funnel shaped holding portions, which are moved against each other in order to catch a foreign matter. After the foreign matter is caught, both holding portions are led very close to each other, thereby firmly holding and even squeezing the foreign matter.

D3 (WO 02/092148) discloses a retrieval catheter comprising a snare tool which has a loop shaped holding member and a straight wire as a second holding member. Both of these holding members are movable relative to each other in order to catch a foreign matter, for which purpose both holding elements must be guided around the foreign matter.

D4 (EP 1 273 268 A1) discloses an intravascular obstruction removing wire having a trapping portion consisting of two branched wire portions connected by filament portions. The trapping portion is deformable in order to catch the obstruction in the blood vessel.

It is the object of the invention to provide a wire for removing foreign matter in a blood vessel having in improved operability, and a medical device using the wire. The object of the invention is achieved by a wire for removing foreign matter in a blood vessel according to claim 1. Advantageous embodiments are carried out according to the dependent claims.

According to the present invention, the positions of holding the foreign matter in the blood vessel can be changed according to the size of the foreign matter, so that the foreign matter can be caught and removed reliably irrespective of the size of the foreign matter.

In the case that each of the first and second holding portions has a looped shape, both the holding portions can enough reach a fine portion in the blood vessel.

In the case that each of the first and second holding portions includes loop wire portions different in size, the foreign matter once caught by the first and second holding portions can be prevented from falling. Accordingly, the foreign matter can be caught and removed more reliably.

In the case that at least one of the first and second holding portions is formed of a radiopaque material, the held condition of the foreign matter by the first and second holding portions can be easily recognized under radioscopy using X rays or the like. Accordingly, the foreign matter can be caught and removed more reliably.

Other objects and features of the invention will be more fully understood from the following detailed description and appended claims when taken with the accompanying drawings.

### BRIEF DESCRIPTION OF THE DRAWINGS

FIG. 1 is a longitudinal sectional view showing a first preferred embodiment of the wire for removing foreign matter in a blood vessel according to the present invention;
FIG. 2 is a longitudinal sectional view showing a condition where the foreign matter is held by the wire shown in FIG. 1;
FIGS. 3A and 3B are a plan view and a side view of retaining means included in the wire shown in FIG. 1, respectively;
FIGS. 4 to 8 are longitudinal sectional views for sequentially illustrating the steps of a using method for the wire shown in FIG. 1;
FIG. 9 is a longitudinal sectional view showing a second preferred embodiment of the wire for removing foreign matter in a blood vessel according to the present invention;
FIG. 10 is a longitudinal sectional view showing a condition where the foreign matter is held by the wire shown in FIG. 9;
FIG. 11 is a longitudinal sectional view showing a third preferred embodiment of the wire for removing foreign matter in a blood vessel according to the present invention; and
FIG. 12 is a longitudinal sectional view showing a condition where the foreign matter is held by the wire shown in FIG. 11.

### DETAILED DESCRIPTION OF THE PREFERRED EMBODIMENTS

Some preferred embodiments of the present invention will now be described in detail with reference to the attached drawings.

### (First Preferred Embodiment)

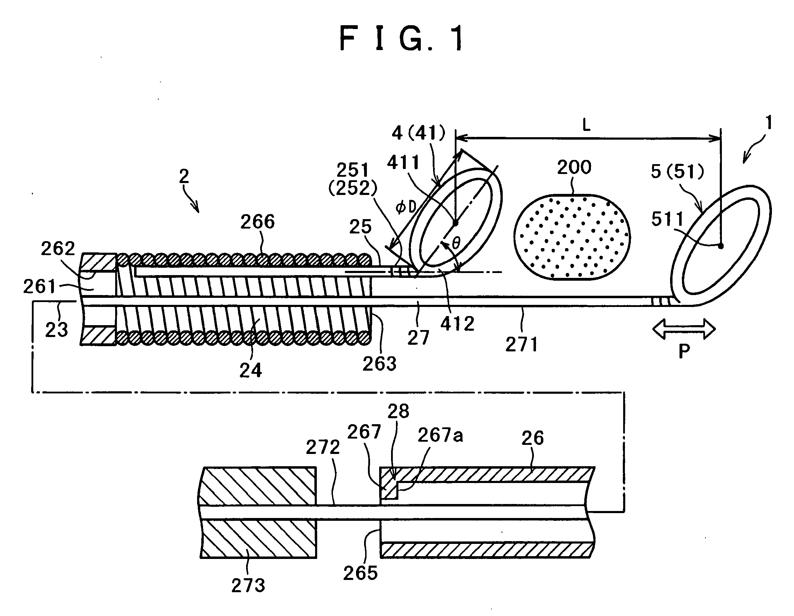

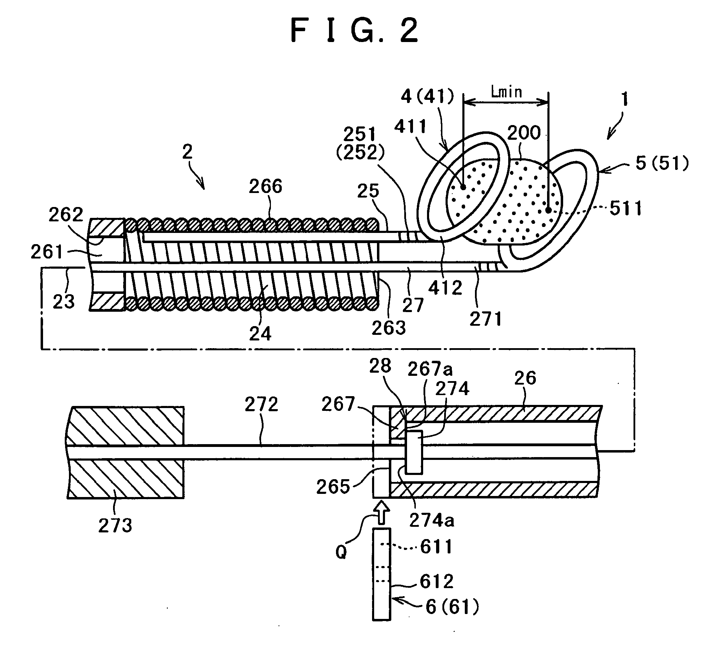

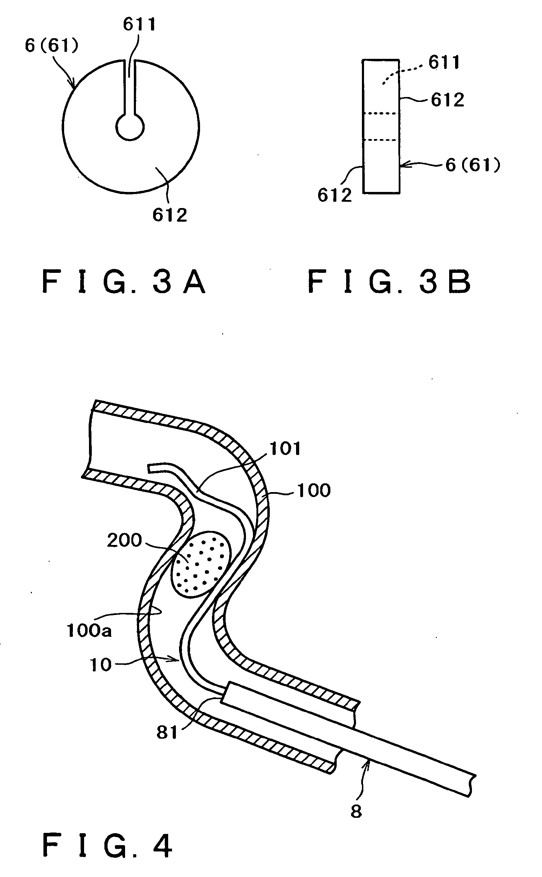

FIG. 1 is a longitudinal sectional view showing a first preferred embodiment of the wire for removing foreign matter in a blood vessel according to the present invention. FIG. 2 is a longitudinal sectional view showing a condition where the foreign matter is held by the wire shown in FIG. 1. FIGS. 3A and 3B are a plan view and a side view of retaining means included in the wire shown in FIG. 1, respectively. FIGS. 4 to 8 are longitudinal sectional views for sequentially illustrating the steps of a using method for the wire shown in FIG. 1.

In the following description, the terms of "base end (proximal end)" and "front end (distal end)" mean the left side and the right side as viewed in FIGS. 1 and 2, respectively, and mean the right side and the left side as viewed in FIGS. 4 to 8, respectively.

Referring to FIG. 1, reference numeral 1 generally denotes the wire according to the first preferred embodiment. The wire 1 is designed to sandwich (catch) and remove the foreign matter (which will be hereinafter referred to as "embolus 200") causing an embolism such as a thrombus and a blood clot in a blood vessel 100.

The wire 1 includes an elongated wire body 2, a first holding portion 4 and a second holding portion 5 each provided at the front end of the wire body 2, distance limiting means 28, and retaining means 6.

The second holding portion 5 is located on the front end side (distal side) relative to the first holding portion 4, and is movable relative to the first holding portion 4 in the longitudinal direction of the wire body 2 (in the direction shown by the arrow P in FIG. 1). In other words, the second holding portion 5 is movable toward or away from the first holding portion 4.

In the following description, the condition where the embolus 200 is held by the wire 1 will be referred to as "held condition".

The components of the wire 1 will now be described individually.

The wire body 2 shown in FIG. 1 (also shown in FIG. 2) includes a tubular member 26 and a second linear member 27 inserted in the inside space (through hole 261) of the tubular member 26, and has suitable rigidity and elasticity (flexibility) over the length.

A coil 266 is provided at (connected to) the front end of the tubular member 26. The coil 266 functions to give further elasticity (flexibility) to the front end portion of the tubular member 26 (inclusive of the coil 266).

A first linear member 25 is fixed to the front end portion of an inner circumferential surface 262 defining the through hole 261 of the tubular member 26 (the inner circumferential surface of the coil 266) so as to extend in the longitudinal direction of the tubular member 26. Accordingly, the displacement of a loop wire portion 41 to be hereinafter described (the first holding portion 4) from the tubular member 26 in its longitudinal direction can be prevented. The first linear member 25 has a front end portion 251 projecting from a front end opening 263 of the tubular member 26.

A fixing method for the first linear member 25 to the tubular member 26 is not especially limited. For example, welding such as brazing and bonding using an adhesive may be adopted.

As shown in FIG. 1, the second linear member 27 is movable relative to the tubular member 26. The second linear member 27 has a front end portion 271 projecting from a front end opening 263 of the tubular member 26, and has a base end portion 272 projecting from a base end opening 265 of the tubular member 26.

The base end portion 272 of the second linear member 27 is provided with an operating member 273 for moving (operating) the second linear member 27 in its longitudinal direction.

Accordingly, the second linear member 27 can be easily grasped in operating the same, so that it can be easily operated.

The materials of these components (members) of the wire body 2 are not especially limited, but various metal materials or various plastics may be used solely or in combination.

The length of the wire body 2 (the tubular member 26 (inclusive of the first linear member 25) and the second linear member 27) is dependent on cases such as the position and thickness of the blood vessel 100. For example, the length of the wire body 2 is preferably set to 500 to 4000 mm, more preferably 1500 to 2200 mm.

The outer diameter of the wire body 2 (the tubular member 26) is dependent on cases such as the position and thickness of the blood vessel 100. For example, the average outer diameter of the wire body 2 is preferably set to 0.1 to 2.0 mm, more preferably 0.25 to 0.9 mm.

Preferably, the wire body 2 (the tubular member 26) has a relatively hard first portion formed on the base side, a relatively soft third portion formed on the front side, and a second portion having variable flexibility formed between the first portion and the third portion. In other words, the wire body 2 preferably has variable rigidity (flexural rigidity, torsional rigidity, etc.) gradually decreasing from the base end toward the front end. Accordingly, the operation on the base side of the wire body 2 can be reliably transmitted to the front end portion 24 of the wire body 2. Further, the movability of the wire body 2 in the blood vessel 100 and the operability of the wire body 2 at a bent portion of the blood vessel 100 can be improved. Further, the flexibility of the front end portion 24 can be improved to thereby prevent damage to the blood vessel 100. Thus, the torque transmittability, pushability, and kink resistance (bending resistance) of the wire body 2 can be maintained and higher safety can also be ensured.

A coating layer may be formed on the outer surface of the wire body 2 (the tubular member 26 (inclusive of the first linear member 25)) to reduce a frictional resistance between the outer surface of the wire body 2 and the inner surface of a catheter 8 to be hereinafter described. Accordingly, the wire body 2 can be more smoothly inserted into and withdrawn from the catheter 8. Examples of this coating layer include a coating layer formed of fluororesin such as polytetrafluoroethylene (Teflon coat ("Teflon" is a registered trademark)) and a hydrophilic polymer coat having lubricity in wetting.

Further, a similar coating layer may be formed on the outer surface of the second linear member 27 to reduce a frictional resistance between the outer surface of the second linear member 27 and the inner circumferential surface 262 of the tubular member 26. Accordingly, a similar effect can be obtained.

As shown in FIG. 1 (also shown in FIG. 2), the first holding portion 4 is formed by a loop wire portion 41 having a looped shape curved in a single direction and present in one plane.

The loop wire portion 41 (the first holding portion 4) is provided (fixed) at the front end of the first linear member 25 in such a manner that the center 411 of the loop wire portion 41 is offset from the axis 23 of the wire body 2 and that the plane in which the loop wire portion 41 is present is inclined with respect to the axis 23 of the wire body 2.

As shown in FIG. 1 (also shown in FIG. 2), the second holding portion 5 is formed by a loop wire portion 51 similar to the loop wire portion 41 of the first holding portion 4.

The loop wire portion 51 (the second holding portion 5) is provided (fixed) at the front end of the second linear member 27 in such a manner that the center 511 of the loop wire portion 51 is offset from the axis 23 of the wire body 2 and that the plane in which the loop wire portion 51 is present is inclined with respect to the axis 23 of the wire body 2.

With the above configuration of the first holding portion 4 and the second holding portion 5, the embolus 200 can be held (caught) more reliably.

Further, each of the first and second holding portions 4 and 5 is formed so as to have a simple looped shape, so that both the holding portions 4 and 5 can enough reach a fine portion (bent portion) in the blood vessel 100. In other words, the first and second holding portions 4 and 5 can be suppressed in size, so that both the holding portions 4 and 5 can enough reach a fine portion (bent portion) in the blood vessel 100.

The loop wire portions 41 and 51 have substantially the same configuration, so the loop wire portion 41 will now be described representatively.

A fixing method for the loop wire portion 41 to the first linear member 25 is not especially limited. For example, the base end portion 412 of the loop wire portion 41 may be fixed to the front end portion 251 of the first linear member 25 by braiding (wrapping), welding such as brazing, or bonding using an adhesive.

In this preferred embodiment, the front end portion 251 of the first linear member 25 is provided with a coil 252 for covering a fixed portion (brazed portion) of the loop wire portion 41 to the first linear member 25. The outer surface of the coil 252 is smoothed to thereby obtain higher safety. The coil 252 is preferably formed by winding a platinum wire or the like. In the case that the coil 252 is formed of a radiopaque material such as platinum, the coil 252 functions as means for visually recognizing the position of the loop wire portion 41 (the first holding portion 4) under radioscopy. This means is also provided for the second holding portion 5.

As mentioned above, the loop wire portion 41 shown in FIG. 1 is tilted up (inclined) with respect to the wire body 2 (the first linear member 25). The angle of inclination of the loop wire portion 41 with respect to the axis 23 of the wire body 2 (with respect to the first linear member 25) (this angle being shown by θ in FIG. 1) is not especially limited. For example, in the case of catching (recovering) a relatively hard embolus 200 (e.g., white thrombus) sticking to the inner wall 100a of the blood vessel 100, the angle θ is preferably set to 45 to 90 degrees, more preferably 45 to 60 degrees.

The loop diameter of the loop wire portion 41 (this diameter being shown by D in FIG. 1) is not especially limited. For example, in the case of catching an embolus 200 (thrombus) present in a cerebral blood vessel, the loop diameter D is preferably set to 1 to 20 mm, more preferably 2 to 4 mm.

The outer diameter (thickness) of the loop wire portion 41 is not especially limited. For example, in the case of catching an embolus 200 (thrombus) present in a cerebral blood vessel, the outer diameter of the loop wire portion 41 is preferably set to 0.02 to 0.20 mm, more preferably 0.04 to 0.12 mm.

The loop wire portion 41 (the first holding portion 4) is preferably formed of a radiopaque material. Examples thereof include but are not limited to gold, platinum, platinum-iridium alloy, tungsten, tantalum, palladium, lead, silver, and any alloys and compounds containing at least one kind of these elements.

By using such a radiopaque material as the material of the loop wire portion 41, the held condition of the embolus 200 by the loop wire portion 41 can be easily recognized under radioscopy using X rays or the like.

Further, the material of the loop wire portion 41 preferably includes an alloy showing anelasticity (inclusive of an alloy showing superelasticity or pseudoelasticity (which will be hereinafter referred to as "superelastic alloy")) in a living body (at at least a living body temperature (near 37°C)).

The alloy showing anelasticity (which will be hereinafter referred to as "anelastic alloy") includes any such alloys showing any shapes of a stress-strain curve due to tension, wherein transformation points such as As, Af, Ms, and Mf may or may not be remarkably measured, and these alloys are largely deformed (distorted) by application of a stress and restore an original shape by removal of the stress.

The anelastic alloy includes the superelastic alloy as mentioned above. Preferred examples of the superelastic alloy include Ni-Ti based alloys such as a Ni-Ti alloy containing 49 to 52 at% of Ni, a Cu-Zn alloy containing 38.5 to 41.5 wt% of Zn, a Cu-Zn-X alloy containing 1 to 10 wt% of X (X is at least one kind of Be, Si, Sn, Al, and Ga), and a Ni-Al alloy containing 36 to 38 at% of Al. The most preferable one of these examples is the Ni-Ti based alloys.

By using such anelastic alloy as the material of the loop wire portion 41, the loop wire portion 41 can obtain sufficient flexibility and restorability against bending. Accordingly, even when the loop wire portion 41 is repeatedly deformed, unrecoverable bending of the loop wire portion 41 can be prevented owing to its excellent restorability.

By operating the operating member 273 for the wire body 2 (the second linear member 27) in the wire 1, the second linear member 27 is moved to thereby move the loop wire portion 51.

Accordingly, the loop wire portion 51 (the second holding portion 5) can be moved toward or away from the loop wire portion 41 (the first holding portion 4), so that the distance (the length shown by L in FIG. 1) between the loop wire portions 41 and 51 (between the first and second holding portions 4 and 5) can be varied. The embolus 200 in the blood vessel 100 is held by reducing the distance L (see FIG. 2).

Thus, the distance L between the loop wire portions 41 and 51 can be changed according to the size of the embolus 200 (the length of the embolus 200 in the horizontal direction as viewed in FIG. 2), thereby sandwiching the embolus 200 between the loop wire portions 41 and 51. Accordingly, the embolus 200 once caught (held) can be prevented from falling from the loop wire portions 41 and 51, thereby reliably holding and removing the embolus 200.

As shown in FIG. 2, the distance limiting means 28 functions to limit the minimum distance (the length shown by Lmin in FIG. 2) between the loop wire portion 41 (the first holding portion 4) and the loop wire portion 51 (the second holding portion 5) in the held condition.

As the distance limiting means 28, a projecting portion 267 radially projecting from the inner circumferential surface 262 of the tubular member 26 is formed near the base end opening 265 of the tubular member 26, and a diameter enlarged portion 274 radially projecting from the outer circumferential surface of the second linear member 27 is formed at a portion of the second linear member 27 present in the through hole 261 of the tubular member 26. In other words, the distance limiting means 28 consists of the projecting portion 267 of the tubular member 26 and the diameter enlarged portion 274 of the second linear member 27.

For example, the diameter enlarged portion 274 may be provided by a ringlike member fixed to the second linear member 27.

In the held condition obtained by moving the second linear member 27 relative to the tubular member 26 toward the base end, the diameter enlarged portion 274 of the second linear member 27 comes into abutment against the projecting portion 267 of the tubular member 26. More specifically, the diameter enlarged portion 274 has a base end surface 274a adapted to abut against a front end surface 267a of the projecting portion 267, thereby preventing excess movement of the second linear member 27 toward the base end. In this abutting condition of the diameter enlarged portion 274 against the projecting portion 267, the distance L is maintained at the minimum distance Lmin.

By providing the distance limiting means 28, a holding force (maximum holding force) of holding the embolus 200 between the loop wire portions 41 and 51 can be controlled (suppressed) to thereby prevent breaking of the embolus 200 due to an excess holding force of the loop wire portions 41 and 51.

Further, it is also possible to prevent that the second holding portion 5 may be moved beyond the first holding portion 4 toward the base end and that the second holding portion 5 may be drawn into the tubular member 26.

The minimum distance Lmin is not especially limited. For example, in the case of catching the embolus 200 (thrombus) in a cerebral blood vessel, the minimum distance Lmin is preferably set to 2 to 50 mm, more preferably 2 to 10 mm.

The diameter enlarged portion 274 may be adjusted in position with respect to the second linear member 27. Accordingly, the minimum distance Lmin is adjustable in this case.

Therefore, the minimum distance Lmin can be set according to the size (length) of the embolus 200.

The retaining means 6 functions to retain the held condition of the embolus 200 between the loop wire portions 41 and 51 (see FIG. 2).

As shown in FIGS. 3A and 3B, the retaining means 6 is provided by a C-shaped clip 61 having a cutout 611 and opposite end surfaces 612.

As shown in FIG. 2, the clip 61 is fixed to the second linear member 27 in such a manner that the front end surface 612 is slid in contact with the base end of the tubular member 26 and that the cutout 611 is fitted to the second linear member 27 (in the direction shown by the arrow Q in FIG. 2).

By providing the clip 61 (the retaining means 6), unintentional forward movement of the loop wire portion 51 in the held condition can be prevented to thereby retain the distance L. Accordingly, it is possible to prevent the embolus 200 in the held condition from falling from the loop wire portions 41 and 51.

The material of the clip 61 is not especially limited, but various metal materials or plastics may be used solely or in combination.

The inner diameter of the clip 61 is preferably set substantially equal to or slightly smaller than the outer diameter (thickness) of the second linear member 27. Accordingly, the clip 61 can be firmly fixed to the second linear member 27.

Further, the surface of the second linear member 27 at a portion for mounting the clip 61 may be treated so as to increase friction. For example, surface roughening may be performed. Accordingly, a similar effect can be obtained.

The shape of the loop wire portions 41 and 51 is not limited to the looped shape curved in a fixed direction as in this preferred embodiment, but a looped shape curved in plural directions may be adopted.

Reference numeral 9 generally denotes a medical device having the wire 1 and a catheter 8 formed with a lumen 82.

A using method for the wire 1 will now be described in detail.

(1) FIG. 4 shows a condition where the embolus 200 such as a thrombus is present in the blood vessel 100 to block the blood stream. The embolus 200 is pressed on the inner wall 100a of the blood vessel 100 by the blood pressure, so that the embolus 200 is hard to move. Further, the presence of the embolus 200 is recognized under angiography.

The catheter (microcatheter) 8 and a guide wire 10 inserted in the lumen 82 of the catheter 8 are inserted into the blood vessel 100, and a front end portion 101 of the guide wire 10 projecting from a front end opening 81 of the catheter 8 is inserted so as to pass aside the embolus 200 (toward the peripheral side of the blood vessel 100). In other words, the front end portion 101 of the guide wire 10 is passed through a spacing between the embolus 200 and the inner wall 100a of the blood vessel 100 to reach a position beyond the embolus 200. This operation can be performed more easily by using a micro-guidewire having excellent lubricity as the guide wire 10.

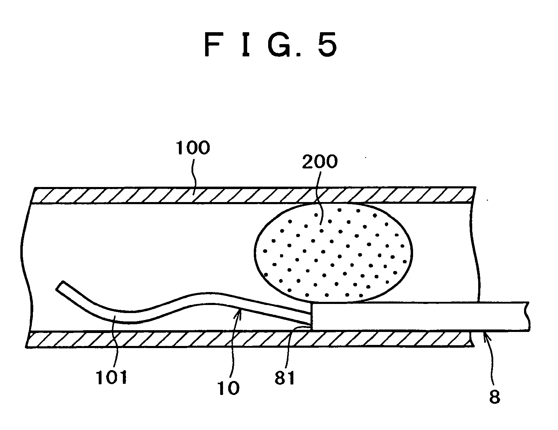

(2) After the front end portion 101 of the guide wire 10 has reached the position beyond the embolus 200, the catheter 8 is advanced over the guide wire 10 to insert the front end portion of the catheter 8 into the spacing between the embolus 200 and the inner wall 100a of the blood vessel 100 as shown in FIG. 5. This operation can be easily performed because the front end portion of the catheter 8 is smoothly inserted into the above spacing along the guide wire 10.

In the conventional treatment, a thrombolytic agent is reversely (retrogradely) passed through the catheter 8 in this condition to accelerate the dissolution of a thrombus. However, doctor's frequent experiences show that there exists a thrombus that cannot be dissolved by a thrombolytic agent and that a long period of time is required for the dissolution of a thrombus. The present invention is useful also in such a case.

(3) The guide wire 10 is next withdrawn from the catheter 8 in the condition shown in FIG. 5, and the wire 1 is next inserted (accommodated) into the lumen 82 of the catheter 8.

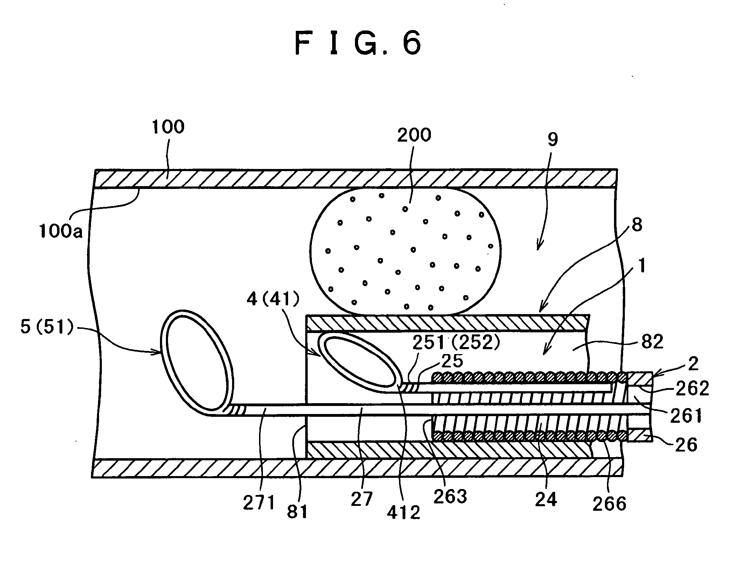

(4) As shown in FIG. 6, the loop wire portion 51 is projected (exposed) from the front end opening 81 of the catheter 8. At this time, the loop wire portion 51 is located at a position deeper than the embolus 200 (on the peripheral side of the blood vessel 100), and the loop forming plane of the loop wire portion 51 is oriented to the embolus 200.

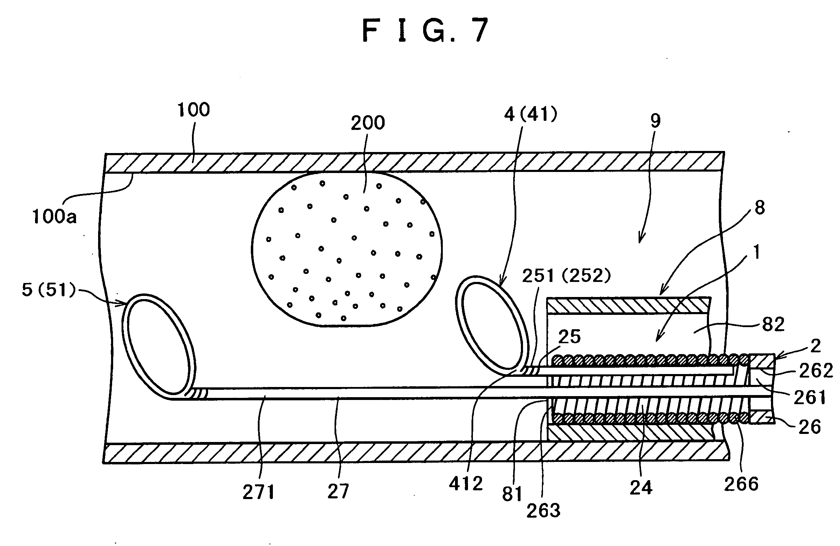

(5) In the next step, the catheter 8 and the tubular member 26 are slightly moved backwardly with the position of the loop wire portion 51 shown in FIG. 6 being maintained. Further, the loop wire portion 41 is next projected (exposed) from the front end opening 81 of the catheter 8 as shown in FIG. 7. At this time, it is visually recognized by angiography or the like that the loop wire portion 51, the embolus 200, and the loop wire portion 41 are arranged in this order from the front side in the blood vessel 100.

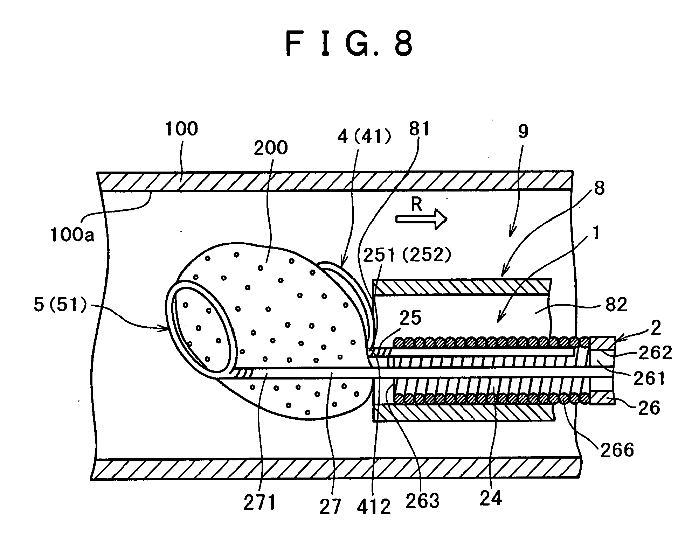

(6) In the next step, the operating member 273 for the second linear member 27 is operated to move the loop wire portion 51 backwardly from the condition shown in FIG. 7, thereby separating the embolus 200 from the inner wall 100a of the blood vessel 100. Then, the loop wire portion 51 is further moved backwardly to sandwich (catch) the embolus 200 between the loop wire portions 41 and 51 as shown in FIG. 8.

(7) In the condition where the embolus 200 is held by the loop wire portions 41 and 51 as shown in FIG. 8, the wire 1 (the tubular member 26 and the second linear member 27) is pulled backwardly (in the direction shown by the arrow R in FIG. 8) to thereby locate the embolus 200 near the front end opening 81 of the catheter 8. Accordingly, the embolus 200 is kept in contact with the front end of the catheter 8, so that the embolus 200 can be held (retained) more reliably.

(8) The wire 1 and the catheter 8 are next withdrawn with the above held condition of the embolus 200 being maintained. Accordingly, the embolus 200 is collected in (removed into) a parent guiding catheter or sheath introducer (not shown).

The step (7) mentioned above may be omitted. That is, just after the embolus 200 is held by the loop wire portions 41 and 51, the wire 1 and the catheter 8 may be withdrawn to remove the embolus 200.

In the case that the loop forming plane of the loop wire portion 51 is not oriented to the embolus 200 in the step (4), it is preferable to adjust the loop forming plane of the loop wire portion 51 at the position shown in FIG. 6.

Further, in this case, it is preferable to give a contrast agent from the base end of the catheter 8 and operate the operating member 273 for the second linear member 27 as visually recognizing the embolus 200 and the loop wire portion 51.

### (Second Preferred Embodiment)

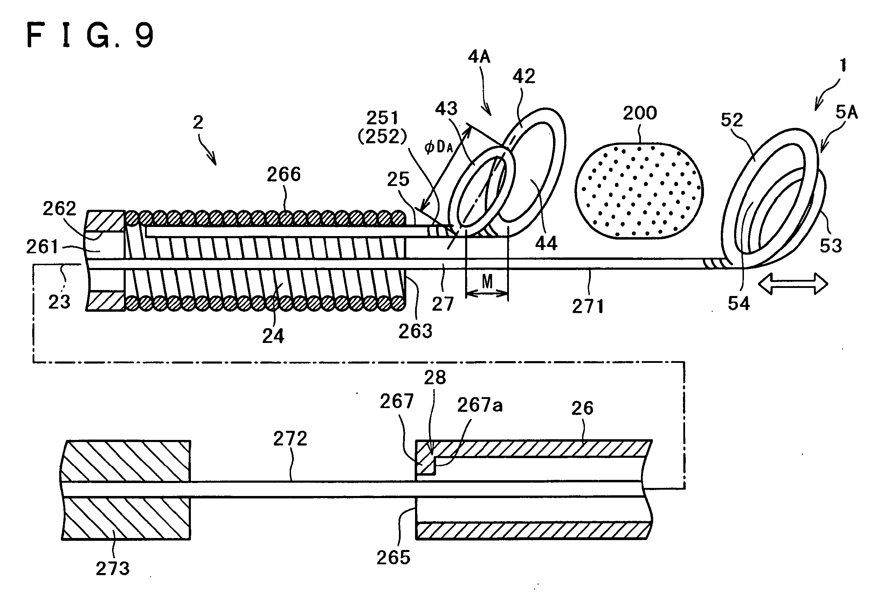

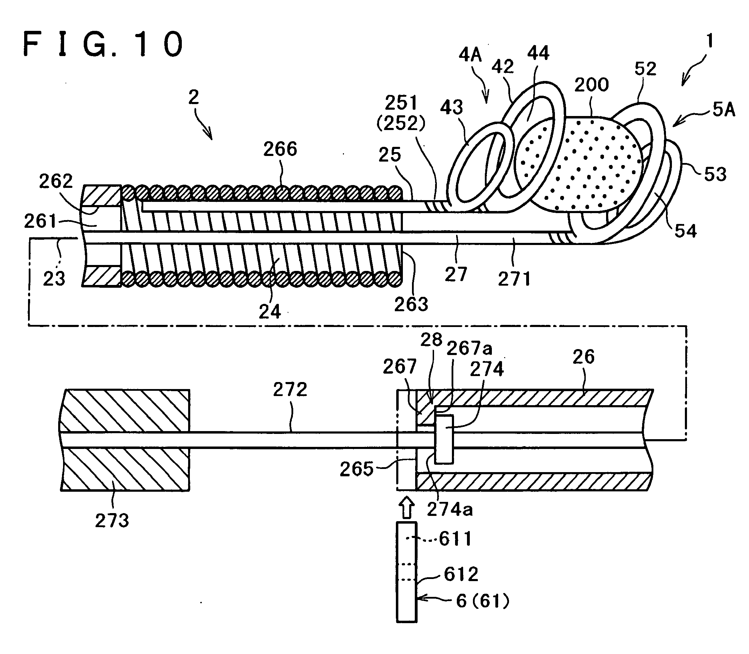

FIG. 9 is a longitudinal sectional view showing a second preferred embodiment of the wire for removing foreign matter in a blood vessel according to the present invention. FIG. 10 is a longitudinal sectional view showing a condition where the foreign matter is held by the wire shown in FIG. 9.

While the second preferred embodiment will now be described with reference to FIGS. 9 and 10, only a difference between the first preferred embodiment and the second preferred embodiment will be focused and the description of similar points will be omitted herein.

In the following description, the terms of "base end" and "front end" mean the left side and the right side as viewed in FIGS. 9 and 10, respectively.

The second preferred embodiment is similar to the first preferred embodiment except the configuration of the first and second holding portions.

As shown in FIG. 9 (also shown in FIG. 10), the wire 1 according to the second preferred embodiment includes a first holding portion 4A and a second holding portion 5A. The first holding portion 4A includes a first loop wire portion 42 and a second loop wire portion 43. The second holding portion 5A includes a first loop wire portion 52 and a second loop wire portion 53.

The first loop wire portions 42 and 52 are similar to the loop wire portions 41 and 51 in the first preferred embodiment, respectively.

The second loop wire portions 43 and 53 are substantially similar in shape to the first loop wire portions 42 and 52, respectively. However, the second loop wire portions 43 and 53 are smaller in loop diameter (size) than the first loop wire portions 42 and 52, respectively.

In the first holding portion 4A, the first loop wire portion 42 is located on the front end side (distal side) relative to the second loop wire portion 43 along the first linear member 25 (the wire body 2).

In the second holding portion 5A, the second loop wire portion 53 is located on the front end side (distal side) relative to the first loop wire portion 52 along the second linear member 27 (the wire body 2).

With the above configuration of the first and second holding portions 4A and 5A, a base end portion of the embolus 200 can be accommodated in a space 44 defined in the first holding portion 4A (between the first loop wire portion 42 and the second loop wire portion 43) in the held condition (see FIG. 10). Similarly, a front end portion of the embolus 200 can be accommodated in a space 54 defined in the second holding portion 5A (between the first loop wire portion 52 and the second loop wire portion 53) in the held condition (see FIG. 10). Accordingly, the embolus 200 can be held more reliably (firmly). In other words, it is possible to more reliably prevent the embolus 200 from falling from the first and second holding portions 4A and 5A.

Since the second loop wire portions 43 and 53 have substantially the same configuration, only the second loop wire portion 43 will now be described representatively.

The loop diameter of the second loop wire portion 43 (this diameter being shown by D_{A} in FIG. 9) is not especially limited. For example, in the case of catching an embolus 200 (thrombus) present in a cerebral blood vessel, the loop diameter D_{A} is preferably set to 1 to 15 mm, more preferably 1 to 3 mm.

The outer diameter (thickness) of the second loop wire portion 43 is not especially limited. For example, in the case of catching an embolus 200 (thrombus) present in a cerebral blood vessel 100, the outer diameter of the second loop wire portion 43 is preferably set to 0.02 to 0.20 mm, more preferably 0.04 to 0.12 mm.

The distance between the first loop wire portion 42 and the second loop wire portion 43 (this distance being shown by M in FIG. 9) is not especially limited. For example, in the case of catching an embolus 200 (thrombus) present in a cerebral blood vessel, the distance M is preferably set to 1 to 10 mm, more preferably 1 to 3 mm.

### (Third Preferred Embodiment)

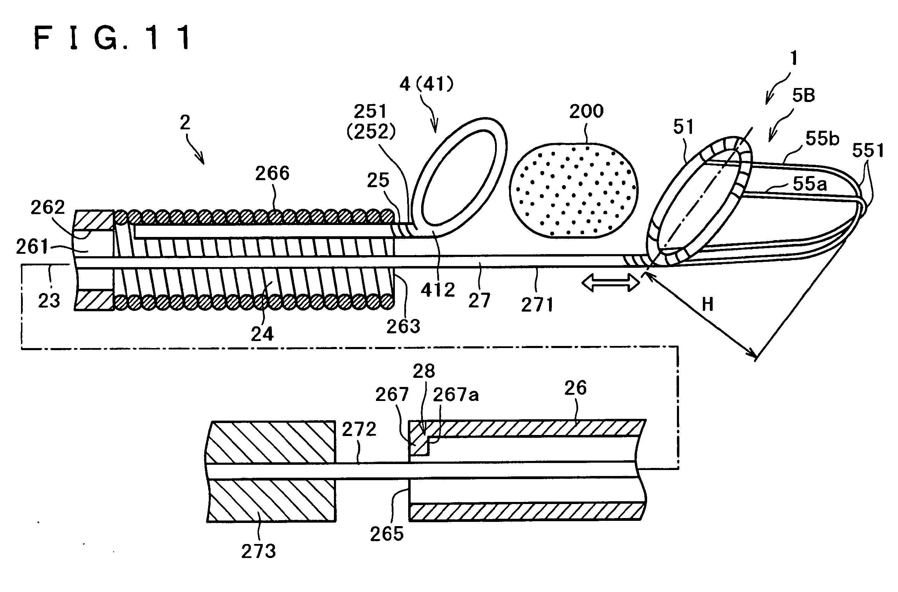

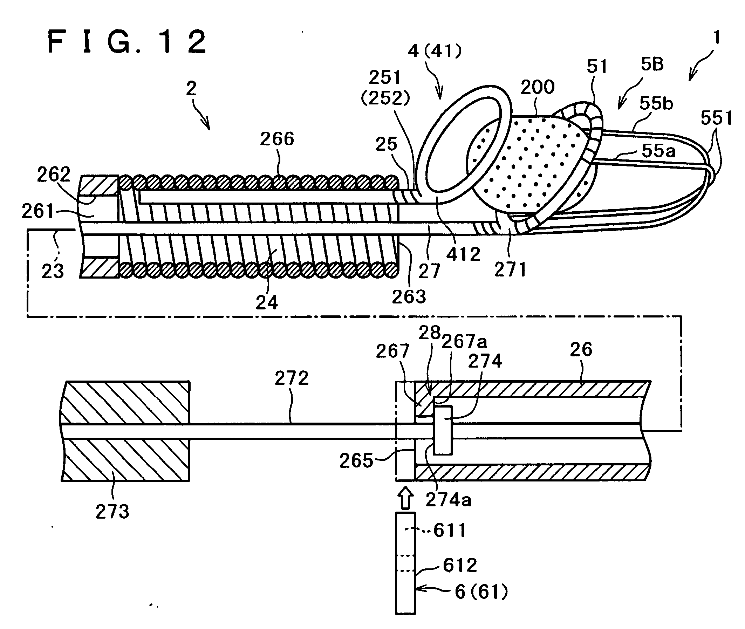

FIG. 11 is a longitudinal sectional view showing a third preferred embodiment of the wire for removing foreign matter in a blood vessel according to the present invention. FIG. 12 is a longitudinal sectional view showing a condition where the foreign matter is held by the wire shown in FIG. 11.

While the third preferred embodiment will now be described with reference to FIGS. 11 and 12, only a difference between the first preferred embodiment and the third preferred embodiment will be focused and the description of similar points will be omitted herein.

In the following description, the terms of "base end" and "front end" mean the left side and the right side as viewed in FIGS. 11 and 12, respectively.

The third preferred embodiment is similar to the first preferred embodiment except the configuration of the second holding portion.

As shown in FIG. 11 (also shown in FIG. 12), the wire 1 according to the third preferred embodiment includes a first holding portion 4 and a second holding portion 5B. The first holding portion 4 is similar to that in the first preferred embodiment. The second holding portion 5B includes a loop wire portion 51 similar to that shown in FIG. 1 and a plurality of (e.g., two) filament portions 55a and 55b fixed to the loop wire portion 51 by twisting.

Each of the filament portions 55a and 55b is fixed to the loop wire portion 51 at two symmetrical positions (diametrically opposite positions).

Each of the filament portions 55a and 55b has an arch shape curved toward the front end so as to form a vertex portion 551. These filament portions 55a and 55b intersect each other near the vertex portions 551. The intersection of the filament portions 55a and 55b means that the filament portions 55a and 55b are disposed close to each other and not always in contact with each other. Further, even when the filament portions 55a and 55b are in contact with each other at the intersecting position, the intersecting position may be movable.

With the configuration that the filament portions 55a and 55b intersect each other, it is possible to prevent the embolus 200 once caught by the loop wire portion 51 from falling from the front side thereof (see FIG. 12).

Thus, the embolus 200 can be caught (removed) more reliably.

The distance between the loop wire portion 51 and each vertex portion 551 (this distance being shown by H in FIG. 11) is not especially limited. For example, in the case of catching an embolus 200 (thrombus) present in a cerebral blood vessel, the distance H is preferably set to 3 to 20 mm, more preferably 3 to 10 mm.

The outer diameter (thickness) of each of the filament portions 55a and 55b is not especially limited. For example, in the case of catching an embolus 200 (thrombus) present in a cerebral blood vessel, the outer diameter of each of the filament portions 55a and 55b is preferably set to 0.01 to 0.20 mm, more preferably 0.04 to 0.1 mm. Further, the number of the filament portions 55a and 55b is not especially limited, but it is preferably set to 2 to 8, more preferably 2 to 4.

Further, while each of the filament portions 55a and 55b is fixed to the loop wire portion 51 at two symmetrical positions in this preferred embodiment, the fixing positions of each filament portion to the loop wire portion 51 may be unsymmetrical.

Further, while the two filament portions 55a and 55b are fixed to the loop wire portion 51 in this preferred embodiment, three or more filament portions may be fixed.

Further, while the filament portions 55a and 55b are fixed to the loop wire portion 51 by twisting in this preferred embodiment, any other fixing methods including braiding (wrapping), welding such as brazing, and bonding using an adhesive may be adopted.

While the specific preferred embodiments of the present invention have been described, the present invention is not limited to these preferred embodiments, but the components of the wire and the medical device may be replaced by any arbitrary parts having similar functions. Further, any arbitrary parts may be added.

The wire according to the present invention may be configured by combining at least two arbitrary configurations (features) of the above preferred embodiments.

For example, in the wire according to the first preferred embodiment, the second holding portion may include two loop wire portions as in the second preferred embodiment.

In the wire according to the third preferred embodiment, the first holding portion may include two loop wire portions as in the second preferred embodiment.

In the wire according to the second preferred embodiment, each of the first and second holding portions may include three or more loop wire portions.

While each of the first and second holding portions is formed of a radiopaque material in the above preferred embodiments, either the first holding portion or the second holding portion may be formed of a radiopaque material.

While each of the first and second holding portions is formed of anelastic alloy in the above preferred embodiments, either the first holding portion or the second holding portion may be formed of anelastic alloy.

In the case that the first holding portion (the second holding portion) is formed of anelastic alloy, the whole of the first holding portion may be formed of anelastic alloy, or a part of the first holding portion may be formed of anelastic alloy.

The surfaces of the first and second holding portions may be provided with slip prevention means, so as to more reliably hold the embolus sandwiched between the first and second holding portions. For example, the slip prevention means may be provided by a coating of elastic material such as rubber having a relatively high coefficient of friction, or minute irregularities (inclusive of a rough surface) formed by sand blasting or the like.

While the invention has been described with reference to specific embodiments, the description is illustrative and is not to be construed as limiting the scope of the invention. Various modifications and changes may occur to those skilled in the art without departing from the spirit and scope of the invention as defined by the appended claims.

Disclosed herein is a wire for removing foreign matter such as an embolus in a blood vessel. The wire includes a flexible elongated wire body and first and second holding portions provided at the front end of the wire body in spaced relationship with each other. The first and second holding portions are movable relative to each other in a longitudinal direction of the wire body. In operation, the first and second holding portions are moved relative to each other to reduce the distance between the first and second holding portions, thereby holding the foreign matter therebetween. Accordingly, the foreign matter in the blood vessel can be caught and removed reliably irrespective of the size of the foreign matter.

## Claims

1. A wire (1) for removing foreign matter (200) in a blood vessel, said wire (1) comprising:
a flexible elongated wire body (2); and
a first holding portion (4) and a second holding portion (5) provided at the front end of said wire body (2) in spaced relationship with each other, said first and second holding portions (4, 5) being movable relative to each other in a longitudinal direction of said wire body;
whereby said first and second holding portions (4, 5) are movable relative to each other to reduce the distance between said first and second holding portions (4, 5), thereby holding said foreign matter (200) therebetween,
**characterized in that**
said wire body (2) comprises a tubular member (26) to which a first linear member (25) is fixed and said first holding portion (4) is provided at the front end of said first linear member (25), and
a second linear member (27) is inserted through said tubular member (26) so as to be movable relative to said tubular member (26); and
said second holding portion (5) is provided at the front end of said second linear member (27).

2. A wire (1) for removing foreign matter (200) in a blood vessel according to claim 1, wherein each of said first and second holding portions (4, 5) has a center offset from the axis of said wire body.

3. A wire (1) for removing foreign matter (200) in a blood vessel according to claim 1, further comprising an operating member (273) provided at the base end of said second linear member (27) for moving said second linear member (27) in a longitudinal direction thereof.

4. A wire (1) for removing foreign matter in a blood vessel according to any one of claims 1 to 3, wherein each of said first and second holding portions (4, 5) comprises at least one loop wire (41, 51) portion having a looped shape.

5. A wire (1) for removing foreign matter in a blood vessel according to any one of claims 1 to 4, wherein said second holding portion (5) is located on the front end side relative to said first holding portion (4) along said wire body (2);
each of said first and second holding portions (4, 5) comprises a first loop wire portion (42) having a looped shape and a second loop wire portion (43) having a looped shape and smaller in size than said first loop wire portion (42);
said first loop wire portion (42) in said first holding portion is located on the front end side relative to said second loop wire portion (43) in said first holding portion (4) along said wire body (2); and
said second loop wire portion (53) in said second holding portion (5) is located on the front end side relative to said first loop wire portion (52) in said second holding portion (5) along said wire body (2).

6. A wire (1) for removing foreign matter (200) in a blood vessel according to any one claims 1 to 4, wherein said first holding portion (4) comprises a loop wire portion (41) having a looped shape;
said second holding portion (5) comprises a loop wire portion (51) located on the front end side relative to said loop wire portion (41) in said first holding portion (4) and having a looped shape, and a plurality of filament portions (55a, 55b) each fixed to said loop wire portion (51) in said second holding portion (5) at two different positions, said filament portions (55a, 55b) being arranged so as to intersect each other.

7. A wire (1) for removing foreign matter (200) in a blood vessel according to claim 6, wherein said plurality of filament portions (55a, 55b) comprise two filament portions each having an arch shape curved so as to form a vertex portion, said two filament portions (55a, 55b) intersecting each other near said vertex portions;
said vertex portions of said two filament portions (55a, 55b) being located on the front end side relative to said loop wire portion (51) in said second holding portion (5) along said wire body (2).

8. A wire (1) for removing foreign matter (200) in a blood vessel according to any one of claims 1 to 7, wherein said first holding portion (4) and/or said second holding portion (5) are/is formed of a radiopaque material.

9. A wire (1) for removing foreign matter (200) in a blood vessel according to any one of claims 1 to 8, wherein at least a part of said first holding portion (4) and/or said second holding portion (5) is formed of alloy showing anelasticity in a living body.

10. A wire (1) for removing foreign matter (200) in a blood vessel according to any one of claims 1 to 9, further comprising distance limiting means (28) for limiting a minimum distance between said first holding portion (4) and said second holding portion (5) in the condition where said foreign matter (200) is held between said first (4) and second holding portions (5).

11. A wire (1) for removing foreign matter (200) in a blood vessel according to claim 10, wherein said minimum distance is adjustable.

12. A wire (1) for removing foreign matter (200) in a blood vessel according to any one of claims 1 to 11, further comprising retaining means for retaining the condition where said foreign matter is held between said first and second holding portions.

13. A medical device comprising a wire for removing foreign matter (200) in a blood vessel according to any one of claims 1 to 12, and a catheter (8) having a lumen (82) capable of accommodating said wire (1).

## Patentansprüche

1. Draht (1) zum Entfernen von Fremdstoffen (200) in einem Blutgefäß, wobei der Draht (1) aufweist:
einen flexiblen verlängerten Drahtkörper (2); und
einen ersten Halteabschnitt (4) und einen zweiten Halteabschnitt (5), die an dem vorderen Ende des Drahtkörpers (2) in einer beabstandeten Beziehung zueinander angeordnet sind, wobei der erste und der zweite Halteabschnitt (4, 5) relativ zueinander in einer Längsrichtung des Drahtkörpers beweglich sind;
wobei der erste und der zweite Halteabschnitt (4, 5) relativ zueinander beweglich sind, um den Abstand zwischen dem ersten und dem zweiten Halteabschnitt (4, 5) zu reduzieren, wodurch der Fremdkörper (200) dazwischen gehalten wird,
**dadurch gekennzeichnet, dass**
der Drahtkörper (2) ein röhrenförmiges Bauteil (26) aufweist, an dem ein erstes gerades Bauteil (25) befestigt ist, wobei der erste Halteabschnitt (4) an dem vorderen Ende des ersten geraden Bauteils (25) vorgesehen ist, und
ein zweites gerades Bauteil (27) durch das röhrenförmige Bauteil (26) hindurch eingesetzt ist, um relativ zu dem röhrenförmigen Bauteil (26) beweglich zu sein; und
der zweite Halteabschnitt (5) an dem vorderen Ende des zweiten geraden Bauteils (27) vorgesehen ist.

2. Draht (1) zum Entfernen von Fremdstoffen (200) in einem Blutgefäß gemäß Anspruch 1, wobei jeder von dem ersten und dem zweiten Halteabschnitt (4, 5) eine Mitte hat, die von der Achse des Drahtkörpers versetzt ist.

3. Draht (1) zum Entfernen von Fremdstoffen (200) in einem Blutgefäß gemäß Anspruch 1, der ferner ein Betätigungsbauteil (273) aufweist, das an dem Basisende des zweiten geraden Bauteils (27) vorgesehen ist, um das zweite gerade Bauteil (27) in dessen Längsrichtung zu bewegen.

4. Draht (1) zum Entfernen von Fremdstoffen in einem Blutgefäß gemäß einem der Ansprüche 1 bis 3, wobei jeder von dem ersten und dem zweiten Halteabschnitt (4, 5) zumindest einen Drahtschleifenabschnitt (41, 51) aufweist, der eine Schleifenform besitzt.

5. Draht (1) zum Entfernen von Fremdstoffen in einem Blutgefäß gemäß einem der Ansprüche 1 bis 4, wobei sich der zweite Halteabschnitt (5) relativ zu dem ersten Halteabschnitt (4) entlang dem Drahtkörper (2) an der vorderen Endseite befindet;
jeder von dem ersten und dem zweiten Halteabschnitt (4, 5) einen ersten Drahtschleifenabschnitt (42) mit einer Schleifenform und einen zweiten Drahtschleifenabschnitt (43) mit einer Schleifenform aufweist, der in seiner Abmessung kleiner als der erste Drahtschleifenabschnitt (42) ist;
sich der erste Drahtschleifenabschnitt (42) in dem ersten Halteabschnitt relativ zu dem zweiten Drahtschleifenabschnitt (43) in dem ersten Halteabschnitt (4) entlang dem Drahtkörper (2) an der vorderen Endseite befindet; und
sich der zweite Drahtschleifenabschnitt (53) in dem zweiten Halteabschnitt (5) relativ zu dem ersten Drahtschleifenabschnitt (52) in dem zweiten Halteabschnitt (5) entlang dem Drahtkörper (2) an der vorderen Endseite befindet.

6. Draht (1) zum Entfernen von Fremdstoffen (200) in einem Blutgefäß gemäß einem der Ansprüche 1 bis 4, wobei der erste Halteabschnitt (4) einen Drahtschleifenabschnitt (41) aufweist, der eine Schleifenform besitzt;
der zweite Halteabschnitt (5) einen Drahtschleifenabschnitt (51) aufweist, der sich relativ zu dem Drahtschleifenabschnitt (41) bei dem ersten Halteabschnitt (4) an der vorderen Endseite befindet und eine Schleifenform besitzt, und eine Vielzahl an Filamentabschnitten (55a, 55b) aufweist, von denen ein jeder an dem Drahtschleifenabschnitt (51) bei dem zweiten Halteabschnitt (5) an zwei unterschiedlichen Positionen befestigt ist, wobei die Filamentabschnitte (55a, 55b) angeordnet sind, um einander zu kreuzen.

7. Draht (1) zum Entfernen von Fremdstoffen (200) in einem Blutgefäß gemäß Anspruch 6, wobei die Vielzahl von Filamentabschnitten (55a, 55b) zwei Filamentabschnitte aufweisen, von denen ein jeder eine Bogenform aufweist, die gekrümmt ist, um einen Scheitelabschnitt zu bilden, wobei sich die zwei Filamentabschnitte (55a, 55b) einander in der Nähe der Scheitelabschnitte kreuzen;
sich die Scheitelabschnitte der zwei Filamentabschnitte (55a, 55b) relativ zu dem Drahtschleifenabschnitt (51) in dem zweiten Halteabschnitt (5) entlang dem Drahtkörper (2) an der vorderen Endseite befinden.

8. Draht (1) zum Entfernen von Fremdstoffen (200) in einem Blutgefäß gemäß einem der Ansprüche 1 bis 7, wobei der erste Halteabschnitt (4) und/oder der zweite Halteabschnitt (5) aus einem Kontrastmittel ausgebildet sind/ist.

9. Draht (1) zum Entfernen von Fremdstoffen (200) in einem Blutgefäß gemäß einem der Ansprüche 1 bis 8, wobei zumindest ein Teil von dem ersten Halteabschnitt (4) und/oder dem zweiten Halteabschnitt (5) aus einer Legierung ausgebildet ist, die in einem lebenden Körper eine unvollkommene Elastizität aufweist.

10. Draht (1) zum Entfernen von Fremdstoffen (200) in einem Blutgefäß gemäß einem der Ansprüche 1 bis 9, der ferner eine Abstandsbegrenzungseinrichtung (28) aufweist, um einen Minimalabstand zwischen dem ersten Halteabschnitt (4) und dem zweiten Halteabschnitt (5) bei dem Zustand zu begrenzen, bei dem ein Fremdkörper (200) zwischen dem ersten (4) und dem zweiten Halteabschnitt (5) gehalten wird.

11. Draht (1) zum Entfernen von Fremdstoffen (200) in einem Blutgefäß gemäß Anspruch 10, wobei der Minimalabstand einstellbar ist.

12. Draht (1) zum Entfernen von Fremdstoffen (200) in einem Blutgefäß gemäß einem der Ansprüche 1 bis 11, der ferner eine Halteeinrichtung aufweist, um den Zustand zu halten, bei dem der Fremdkörper zwischen dem ersten und dem zweiten Halteabschnitt gehalten wird.

13. Medizinisches Gerät mit einem Draht zum Entfernen von Fremdstoffen (200) in einem Blutgefäß gemäß einem der Ansprüche 1 bis 12 und einem Katheter (8), der ein Lumen (82) aufweist, das dazu imstande ist, den Draht (1) aufzunehmen.

## Revendications

1. Câble (1) permettant de retirer une substance étrangère (200) dans un vaisseau sanguin, ledit câble (1) comprenant:
un corps de câble (2) allongé flexible; et
une première partie de maintien (4) et une deuxième partie de maintien (5) pourvues à l'extrémité avant dudit corps de câble (2) dans une relation où elles sont espacées l'une de l'autre, lesdites première et deuxième parties de maintien (4, 5) étant mobiles l'une par rapport à l'autre dans une direction longitudinale dudit corps de câble;
de sorte que lesdites première et deuxième parties de maintien (4, 5) soient mobiles l'une par rapport à l'autre afin de réduire la distance entre lesdites première et deuxième parties de maintien (4, 5), maintenant ainsi ladite substance étrangère (200) entre elles,
**caractérisé en ce que**
ledit corps de câble (2) comprend un élément tubulaire (26) auquel un premier élément linéaire (25) est fixé et ladite première partie de maintien (4) est pourvue à l'extrémité avant dudit premier élément linéaire (25), et
un deuxième élément linéaire (27) est inséré à travers ledit élément tubulaire (26) de sorte à être mobile par rapport audit élément tubulaire (26); et
ladite deuxième partie de maintien (5) est pourvue à l'extrémité avant dudit deuxième élément linéaire (27).

2. Câble (1) permettant de retirer une substance étrangère (200) dans un vaisseau sanguin selon la revendication 1, dans lequel chacun desdites première et deuxième parties de maintien (4, 5) a un centre décalé de l'axe dudit corps de câble.

3. Câble (1) permettant de retirer une substance étrangère (200) dans un vaisseau sanguin selon la revendication 1, comprenant en plus un élément de manoeuvre (273) pourvu à l'extrémité de base dudit deuxième élément linéaire (27) pour déplacer ledit deuxième élément linéaire (27) dans une direction longitudinale correspondante.

4. Câble (1) permettant de retirer une substance étrangère dans un vaisseau sanguin selon l'une quelconque des revendications 1 à 3, dans lequel chacune desdites première et deuxième parties de maintien (4, 5) comprend au moins une partie de câble en boucle (41, 51) ayant une forme de boucle.

5. Câble (1) permettant de retirer une substance étrangère dans un vaisseau sanguin selon l'une quelconque des revendications 1 à 4, dans lequel ladite deuxième partie de maintien (5) est située du côté extrémité avant par rapport à ladite première partie de maintien (4) le long dudit corps de câble (2);
chacune desdites première et deuxième parties de maintien (4, 5) comprend une première partie de câble en boucle (42) ayant une forme de boucle et une deuxième partie de câble en boucle (43) ayant une forme de boucle et qui a une plus petite taille que ladite première partie de câble en boucle (42);
ladite première partie de câble en boucle (42) dans ladite première partie de maintien est située du côté extrémité avant par rapport à ladite deuxième partie de câble en boucle (43) dans ladite première partie de maintien (4) le long dudit corps de câble (2); et
ladite deuxième partie de câble en boucle (53) dans ladite deuxième partie de maintien (5) est située du côté extrémité avant par rapport à ladite première partie de câble en boucle (42) dans ladite deuxième partie de maintien (5) le long dudit corps de câble (2).

6. Câble (1) permettant de retirer une substance étrangère (200) dans un vaisseau sanguin selon l'une quelconque des revendications 1 à 4, dans lequel ladite première partie de maintien (4) comprend une partie de câble en boucle (41) ayant une forme de boucle;
ladite deuxième partie de maintien (5) comprend une partie de câble en boucle (51) située du côté extrémité avant par rapport à ladite partie de câble en boucle (41) dans ladite première partie de maintien (4) et ayant une forme de boucle, et plusieurs parties de filament (55a, 55b) chacune fixée à ladite partie de câble en boucle (51) dans ladite deuxième partie de maintien (5) à deux positions différentes, lesdites parties de filament (55a, 55b) étant agencées de sorte à se croiser.

7. Câble (1) permettant de retirer une substance étrangère (200) dans un vaisseau sanguin selon la revendication 6, dans lequel plusieurs parties de filament (55a, 55b) comprennent deux parties de filament chacune ayant une forme d'arc incurvée de sorte à former une partie de vertex, lesdites parties de filament (55a, 55b) se croisant près desdites parties de vertex;
lesdites parties de vertex desdites deux parties de filament (55a, 55b) étant situées du côté extrémité avant par rapport à ladite partie de câble en boucle (51) dans ladite deuxième partie de maintien (5) le long dudit corps de câble (2).

8. Câble (1) permettant de retirer une substance étrangère (200) dans un vaisseau sanguin selon l'une quelconque des revendications 1 à 7, dans lequel ladite première partie de maintien (4) et/ou ladite deuxième partie de maintien (5) est/sont formée(s) d'un matériau radio-opaque.

9. Câble (1) permettant de retirer une substance étrangère (200) dans un vaisseau sanguin selon l'une quelconque des revendications 1 à 8, dans lequel au moins une partie de ladite première partie de maintien (4) et/ou de ladite deuxième partie de maintien (5) est formée d'un alliage présentant une propriété d'anélasticité dans un corps vivant.

10. Câble (1) permettant de retirer une substance étrangère (200) dans un vaisseau sanguin selon l'une quelconque des revendications 1 à 9, comprenant en plus un moyen de limitation de distance (28) pour limiter une distance minimale entre ladite première partie de maintien (4) et ladite deuxième partie de maintien (5) lorsque ladite substance étrangère (200) est maintenue entre lesdites première (4) et deuxième (5) parties de maintien.

11. Câble (1) permettant de retirer une substance étrangère (200) dans un vaisseau sanguin selon la revendication 10, dans lequel ladite distance minimale peut être ajustée.

12. Câble (1) permettant de retirer une substance étrangère (200) dans un vaisseau sanguin selon l'une quelconque des revendications 1 à 11, comprenant en plus un moyen de retenue pour retenir la condition où ladite substance étrangère est maintenue entre lesdites première (4) et deuxième (5) parties de maintien.

13. Dispositif médical comprenant un câble permettant de retirer une substance étrangère (200) dans un vaisseau sanguin selon l'une quelconque des revendications 1 à 12, et un cathéter (8) ayant un lumen (82) capable de loger ledit câble (1).
